Europäisches Patentamt

European Patent Office

Office européen des brevets

(11) Veröffentlichungsnummer: **0 290 484 B1**

(19)

(12) **EUROPÄISCHE PATENTSCHRIFT**

(45) Veröffentlichungstag der Patentschrift: **16.12.92**

(21) Anmeldenummer: **87906182.8**

(22) Anmeldetag: **17.09.87**

(86) Internationale Anmeldenummer:
**PCT/EP87/00533**

(87) Internationale Veröffentlichungsnummer:
**WO 88/03793 (02.06.88 88/12)**

Die Akte enthält technische Angaben, die nach
dem Eingang der Anmeldung eingereicht wurden
und die nicht in dieser Patentschrift enthalten sind.

(51) Int. Cl.⁵: **A61K 7/09, B65D 81/32**

(54) **VERFAHREN ZUR PARFÜMIERUNG VON DAUERWELL- UND DAUERWELL-FIXIERPRÄPARATEN.**

(30) Priorität: **28.11.86 DE 3640748**

(43) Veröffentlichungstag der Anmeldung:
**17.11.88 Patentblatt 88/46**

(45) Bekanntmachung des Hinweises auf die
Patenterteilung:
**16.12.92 Patentblatt 92/51**

(84) Benannte Vertragsstaaten:
**FR GB NL**

(56) Entgegenhaltungen:
**DE-A- 2 644 692     DE-A- 3 138 142**
**DE-B- 1 136 057     GB-A- 2 066 310**
**US-A- 3 143 476     US-A- 4 548 811**

(73) Patentinhaber: **GOLDWELL AKTIENGESELL-**
**SCHAFT**
**Zerninstrasse 10-18**
**W-6100 Darmstadt 13(DE)**

(72) Erfinder: **TENNIGKEIT, Jürgen**
**Felsbergstr. 51**
**W-6104 Seeheim 2(DE)**

**Beschreibung**

Die Erfindung betrifft ein Verfahren zum Überdecken des unangenehmen Geruchs von alkalischen, stark reduzierenden Dauerwell- bzw. sauren, stark oxidierenden Dauerwell-Fixier-Präparaten während ihrer Anwendung.

Die heute verwendeten Dauerwell-Präparate weisen zwei Hauptbestandteile als Wirkstoffe auf, nämlich Haarkeratin reduzierende Substanzen, z.B. Thioglykolsäure oder Thioglykolsäureester, und ein Alkalisierungsmittel, wofür in der Regel Ammoniak verwendet wird. Sowohl die reduzierenden Substanzen als auch das Alkalisierungsmittel haben einen unangenehmen Geruch. Die erwähnten Thio-Verbindungen haben beispielsweise einen unangenehmen Schwefelwasserstoff-Geruch und Ammoniak riecht stechend, wobei hinzu kommt, daß die Geruchsträger ausgesprochen flüchtig sind. Bei der Dauerwellbehandlung verbreitet sich deshalb der Geruch des Dauerwell-Präparats im gesamten Salon, wodurch das Wohlbefinden nicht nur der jeweils behandelten Kundin, sondern auch weiterer Kunden und des Personals insgesamt beeinträchtigt wird. Bei speziell geruchsempfindlichen Personen kann dies sogar zu gesundheitlicher Beeinträchtigung führen.

Trotz vieler Versuche der Hersteller ist es bisher nicht in zufriedenstellendem Maße gelungen, den unangenehmen Geruch von Dauerwell-Präparaten entscheidend zu verringern oder zu überdecken, weil die für kosmetische Präparate üblicherweise geeigneten und verwendeten Parfumöle bei längerer Lagerung wegen der Reaktionsfähigkeit der Dauerwellösung nicht stabil sind.

Neben der Unbeständigkeit der Parfumöle sind in Dauerwellösungen auch die zum Löslichmachen der Parfumöle üblichen Lösungsvermittler (z.B. Rizinusöl-Ethoxylate, oxethylierte Sorbitanfettsäure-Ester, Ölsäure- und Stearinsäure-Glycerinester) bei längerer Lagerung instabil; sie hydrolysieren bzw. zersetzen sich im alkalischen Dauerwell-Präparat und verlieren dadurch ihre parfümlösenden Eigenschaften. Das Dauerwell-Präparat wird dann trüb und das Parfumöl setzt sich ab, wodurch seine geruchsüberdeckende Wirkung stark vermindert wird.

Die bei der Dauerwellbehandlung im Friseursalon auftretende Geruchsbelästigung wurde daher bisher als unvermeidbar hingenommen bzw. es wurde versucht, ihr durch entsprechende Lüftungs-Maßnahmen oder auch sogenannte Luftverbesserer entgegenzuwirken. Ähnliches gilt auch für die sauer eingestellten und Sauerstoff abspaltenden, d.h. stark oxidierenden Dauerwell-Fixiermittel. Aus der Literatur (z.B. J 5 8083 607 Ref.Nr. 62085 K/26 CPI-Basis Abstr. Journal 1983 und J 5 7062 217 Ref.Nr. 42221 E/21 CPI-Basis Abstr. Journal 1982) sind Rezepturen für spezielle Dauerwell-Präparate bekannt, welche bei der Anwendung den bei den heute üblicherweise verwendeten Dauerwell-Präparaten auftretenden unangenehmen Geruch nicht aufweisen. Hinweise auf die Möglichkeit, die störende Geruchsbelästigung von Dauerwell-Präparaten unterschiedlicher Rezeptur sowie von Dauerwell-Fixiermitteln grundsätzlich zu vermeiden, sind den genannten Veröffentlichungen jedoch nicht zu entnehmen.

Der Erfindung liegt die Aufgabe zugrunde, ein Verfahren aufzufinden, welches es ermöglicht, die bei der Anwendung der heute üblicherweise verwendeten Dauerwell-Präparate und Dauerwell-Fixiermittel für unvermeidlich gehaltenen Geruchsbelästigungen zu verhindern oder wesentlich zu vermindern.

Erfindungsgemäß wird diese Aufgabe dadurch gelöst, daß das Präparat einerseits und ein Parfumöl, gegebenenfalls im Gemisch mit einem Lösungsvermittler und/oder Dispergiermittel, andererseits portioniert in zwei gegeneinander abgeschlossenen Räumen eines gemeinsamen geruchsdichten Portionsbehälters abgepackt werden, und daß die Trennung zwischen den das Präparat bzw. das Parfumöl und den Lösungsvermittler und/oder das Dispergiermittel enthaltenden Räumen vor der Öffnung des Portionsbehälters unmittelbar vor der Anwendung unterbrochen und das Präparat mit dem Parfumöl und dem Lösungsvermittler und/oder dem Dispergiermittel vermischt wird.

Die geruchsüberdeckende Wirkung des Parfumöls bleibt während der Behandlungsdauer, d.h., bis nach dem Auswaschen des Dauerwell-Präparate aus dem Haar der zu behandelnden Kundin, voll wirksam, so daß es zu keiner Geruchsbelästigung mehr kommen kann.

Es hat sich gezeigt, daß diese Wirkung unabhängig vom chemischen Aufbau bzw. der Konstitution des verwendeten Parfumöls ist, so daß beliebige gewünschte Duftnoten verwirklichbar sind. Darüber hinaus hat sich gezeigt, daß die bisher üblicherweise angewandten Lösungsvermittler für Parfumöl sämtlich verwendbar sind.

Anstelle oder zusätzlich zum Lösungsvermittler kann auch ein Dispergiermittel zusammen mit dem Parfumöl in das Präparat eingebracht werden.

Aus der DE-A 3 138 142 sind Verdünnungsmittel für Dauerwell-Präparate bekannt, die auch ein Parfumöl enthalten.

Von der erfindungsgemäß gestellten Aufgabe, d.h., der Überdeckung des unangenehmen Geruchs von Dauerwell-Präparaten, ist dort an keiner Stelle die Rede, ebensowenig von der Konfektionierung als Zweikomponenten-Präparat.

Die DE-A 2 644 692 betrifft ein Verfahren zur Herstellung von flüssigen haarkosmetischen Konzentraten, das dadurch gekennzeichnet ist, daß man saugfähige gepreßte Watte mit den Konzentraten bis zur teilweisen oder vollständigen Sättigung der Watte tränkt, dieses Dokument offenbart ferner im einleitenden Teil einen aus zwei getrennten Kammern bestehenden, Behälter, wobei die Trennwand zur Vermischung der beiden getrennten Komponenten beseitigt werden kann. Die GB-A 2 066 310 befaßt sich mit einem Dauerwellverfahren, wobei, wie üblich, in einer ersten Phase eine Reduktionslösung auf Basis einer Thio-Verbindung, die darüber hinaus zur Haarkonditionierung ein kationisches Polymer enthält, auf das Haar einwirken gelassen wird, und in einer zweiten Phase eine Neutralisationslösung, die ein Oxidationsmittel und ein anionisches Tensid enthält, in ebenfalls an sich bekannter Weise auf das Haar aufgebracht wird.

Gemäß Anspruch 12 ff. können beide Zusammensetzungen bis zur Anwendung getrennt in einer Zweikomponenten-Verpackung gelagert werden. Dies steht jedoch in keinerlei Zusammenhang mit und gibt keinerlei Hinweis auf die Möglichkeit einer getrennten Lagerung der Parfumöl-Zusammensetzung und deren Zugabe unmittelbar vor der Anwendung zu der jeweiligen Reduktions- oder Neutralisations-Zusammensetzung, vielmehr enthalten nach dieser Druckschrift beide Zusammensetzungen, wenn überhaupt, in der Regel Parfumöle von Anfang an.

Die DE-A 1 136 057 geht von der Aufgabenstellung aus, ein Verfahren zur Herstellung eines wäßrigen, kohlensäurehaltigen Dauerwellmittels zu schaffen, wobei die Lösung der Aufgabe darin besteht, eine sauer reagierende, eine Mercapto-Verbindung enthaltende Komponente (1) in einem Druckgefäß mit einer kohlensaure Salze enthaltenden Komponente (2) in Reaktion treten zu lassen. Das Problem der Geruchsüberdek-kung ist hier an keiner Stelle angesprochen.

Gleiches gilt schließlich auch für die Offenbarung der US-A 3 143 476, die von der Aufgabenstellung ausgeht, Neutralisationsmittel für Dauerwellen zu schaffen, die eine schnelle und vollständige Neutralisierung gewährleisten. Diese Aufgabe wird gelöst durch den Einsatz von 3 bis 18 Prozent Alkali- bzw. Erdalkalibromat und mindestens 0,001 Prozent Eisen in Gegenwart eines Komplexierungsmittels.

Diese Neutralisierungsmittel stellen keine "stark sauren Dauerwell-Fixierpräparate" im Sinne der Erfindung und der Definition des Patentanspruchs dar, sondern liegen im alkalischen Bereich.

Die US-A-4 548 811 erwähnt im einleitenden Teil die konventionellen Methoden zur Maskierung des typischen Geruchs von Dauerwellpräparaten, die darin bestehen, den Präparaten Parfum zuzufügen.

## Beispiel 1

In einem für die getrennte Aufnahme von zwei Komponenten vorgesehenen (bekannten) Behälter, bei dem ohne Öffnen des Behälters durch Manipulation von außen die Trennung zwischen den die Komponenten aufnehmenden Räumen aufgehoben werden und der Behälter selbst dann durch Schütteln zum Mischen der Komponenten verwendet werden kann, wurden ein Dauerwell-Präparat mit der nachstehend als Mischung 1 angegebenen (üblichen) Zusammensetzung und eine Parfumöl-Lösungsvermittler-Mischung 2 getrennt verpackt und längere Zeit gelagert.

Unmittelbar vor einer durchzuführenden Dauerwellbehandlung wurden die Mischungen 1 und 2 im Behälter zusammengebracht und durch Schütteln miteinander vermischt. Der Geruch des aus dem anschließend geöffneten Behälter auf das vorgewaschene und auf Wickler gewickelte Haar einer Versuchsperson aufgetragenen Dauerwell-Präparats wurde während der gesamten Dauerwellbehandlung ausschließlich von dem Parfumöl bestimmt, während der den Dauerwell-Präparaten üblicherweise eigene stechende Ammoniak/Schwefelwasserstoff-Geruch während der gesamten Behandlungsdauer bis zur Nichtwahrnehmbarkeit herabgesetzt war.

Die bei der Dauerwellbehandlung erzielte Wellung des Haares entsprach dabei der mit einem üblichen Dauerwell-Präparat ohne Parfumölzugabe erzielbaren Wellung.

Rezeptur

EP 0 290 484 B1

| Mischung 1 (Dauerwell-Präparat) | |
|---|---|
| Ammoniumthioglykolat, 50%ig | 14,0 g |
| Ammoniak, 25%ig | 0,60 g |
| Ammoniumhydrogencarbonat | 4,0 g |
| $H_2O$ dest. | 80,5 ml |

| Mischung 2 (Parfumöl u. Lösungsvermittler) | |
|---|---|
| Parfumöl | 0,5 g |
| Cremophor RH 40 (BASF) | 0,4 g |

# Beispiel 2

Unter den gleichen Voraussetzungen wie beim Beispiel 1 wurde unmittelbar vor der Anwendung ein sauer eingestelltes und Sauerstoff abspaltendes Dauerwell-Fixierpräparat (Mischung 3) mit einer Parfumöl-Lösungsvermittler-Mischung (Mischung 4) zusammengebracht und durch Schütteln gemischt. Auch hier wurde der unangenehme metallische Geruch des Fixier-Präparats während des Fixiervorgangs vom Geruch des Parfumöls vollständig überdeckt.

Rezeptur

| Mischung 3 (Dauerwell-Fixierpräparat) | | |
|---|---|---|
| Wasserstoffperoxid | 50% | 4,6 g |
| Stabilisierungsmittel | | 0,3 g |
| $H_2O$, dest. | | 76,55 ml |
| O-Phosphorsäure | 85%ig | 0,3 g |

| Mischung 4 (Parfumöl und Lösungsvermittler) | |
|---|---|
| Parfumöl | 0,35 g |
| Cremophor$^R$ RH 40 (BASF) | 0,20 g |
| $H_2O$, dest. | 20,0 ml |

**Patentansprüche**

1. Verfahren zum Überdecken des unangenehmen Geruchs von alkalischen, stark reduzierenden Dauerwell-Präparaten bzw. stark sauren, oxydierenden Dauerwell-Fixierpräparaten während ihrer Anwendung, dadurch gekennzeichnet, daß das Präparat einerseits und ein Parfumöl, gegebenenfalls im Gemisch mit einem Lösungsvermittler und/oder Dispergiermittel, andererseits portioniert in zwei gegeneinander abgeschlossenen Räumen eines gemeinsamen geruchsdichten Portionsbehälters abgepackt werden, und daß die Trennung zwischen den das Präparat bzw. das Parfumöl und den Lösungsvermittler und/oder das Dispergiermittel enthaltenden Räumen vor der Öffnung des Portionsbehälters unmittelbar vor der Anwendung unterbrochen und das Präparat mit dem Parfumöl und dem Lösungsvermittler und/oder dem Dispergiermittel vermischt wird.

**Claims**

1. Method of masking the unpleasant odour of alkaline, strongly reducing permanent wave preparations or

4

strongly acidic, oxidising permanent wave fixing preparations during use, characterised in that, on the one hand, the preparation and, on the other hand, a perfume oil, optionally in admixture with a solubiliser and/or dispersant, are packaged in portions in two chambers of a common odour-tight portion container, which chambers are sealed off from one another, and the partition between the chamber containing the preparation and the chamber containing the perfume oil and the solubiliser and/or the dispersant is broken prior to opening the portion container immediately before use and the preparation is mixed with the perfume oil and the solubiliser and/or the dispersant.

**Revendications**

1. Procédé pour masquer, pendant leur utilisation, l'odeur désagréable de préparations alcalines, fortement réductrices, pour ondulation permanente ou de préparations de fixateurs fortement acides, oxydants, pour ondulation permanente, procédé caractérisé en ce que la préparation, d'une part, et une huile parfumée, éventuellement en mélange avec un adjuvant de dissolution et/ou un dispersant, d'autre part, sont emballées par portions dans deux espaces, séparés l'un de l'autre, d'un récipient commun, étanche aux odeurs, capable de contenir des portions de matière, et en ce que la séparation entre les espaces contenant respectivement la préparation et l'huile parfumée et l'adjuvant de dissolution et/ou le dispersant est rompue avant l'ouverture du récipient, immédiatement avant l'utilisation, et en ce que la préparation est mélangée avec l'huile parfumée et l'adjuvant de dissolution et/ou le dispersant.